# EUROPÄISCHE PATENTSCHRIFT

(11) **EP 2 987 551 B1**
(45) Veröffentlichungstag und Bekanntmachung des Hinweises auf die Patenterteilung: **13.12.2017**
(21) Anmeldenummer: 15178678.7
(22) Anmeldetag: 28.07.2015
(51) Int. Cl.: B01F 11/00, B01F 13/00, B01F 15/00, B01F 15/02, F03G 1/00, B01F 7/00

(54) **AUSTRAGSVORRICHTUNG MIT ELASTISCH ANGETRIEBENEM MISCHER UND VEFAHREN**
DISPENSING DEVICE WITH ELASTICALLY DRIVEN MIXER AND PROCESS
DISPOSITIF DE SORTIE DOTE D'UN MELANGEUR ENTRAINE ELASTIQUEMENT ET PROCÉDÉ

(30) Priorität: 22.08.2014 DE 102014112042
(43) Veröffentlichungstag der Anmeldung: 24.02.2016
(73) Patentinhaber: Heraeus Medical GmbH, 61273 Wehrheim (DE)
(72) Erfinder: Dr. Vogt, Sebastian, 99092 Erfurt (DE); Dr. Kluge, Thomas, 56179 Vallendar (DE)
(74) Vertreter: Heraeus IP

(56) Entgegenhaltungen:
- WO-A1-94/26403
- WO-A1-98/39088
- DE-B3-102009 031 178
- DE-B3-102011 119 371
- DE-B3-102011 119 377
- DE-C2- 19 745 084
- US-A- 4 832 501
- US-A- 5 201 263
- US-A- 5 497 695
- US-A1- 2008 144 428

## Beschreibung

Die Erfindung betrifft eine Mischvorrichtung für Polymethylmethacrylat-Knochenzemente (PMMA-Knochenzemente).

Die Erfindung betrifft ferner ein Vakuummischsystem mit einer solchen Mischvorrichtung und ein Verfahren zum Mischen eines Zements, insbesondere eines PMMA-Knochenzements.

Gegenstand der Erfindung ist somit eine Mischvorrichtung für die Homogenisierung von Zementkomponenten von Pulver-Flüssigkeits-Polymethylmethacrylat-Knochenzementen. Ein weiterer Gegenstand der Erfindung ist ein geschlossenes Vakuummischsystem für die Lagerung, Vermischung und den Austrag von Polymethylmethacrylat-Knochenzement.

Polymethylmethacrylat-(PMMA)-Knochenzemente gehen auf die grundlegenden Arbeiten von Sir Charnley zurück. PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig vorhanden sein können, die später eine Destabilisierung des Knochenzements verursachen können. Aus diesem Grund wird das Vermischen von Knochenzementpulver mit der Monomerflüssigkeit in Vakuummischsystemen bevorzugt, weil durch Mischen im Vakuum Lufteinschlüsse aus dem Zementteig weitgehend entfernt werden und damit eine optimale Zementqualität erreicht wird. Im Vakuum gemischte Knochenzemente haben eine deutlich verringerte Porosität und zeigen daher verbesserte mechanische Eigenschaften. Es wurden eine Vielzahl von Vakuum-Zementiersystemen offen gelegt, von denen exemplarisch folgende genannt sind: US 6,033,105 A, US 5,624,184 A, US 4,671,263 A, US 4,973,168 A, US 5,100,241 A, WO 99/67015 A1, EP 1 020 167 A2, US 5,586,821 A, EP 1 016 452 A2, DE 36 40 279 A1, WO 94/26403 A1, EP 1 005 901 A2, US 5,344,232 A.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche Full-Prepacked-Mischsysteme wurden mit der EP 0 692 229 A1, der DE 10 2009 031 178 B3, der US 5,997,544 A, der US 6,709,149 B1, der DE 698 12 726 T2 und der US 5,588,745 A vorgeschlagen.

Das Patent DE 10 2009 031 178 B3 offenbart ein geschlossenes Vakuummischsystem mit einem zweiteiligen Austragskolben zum Verschluss einer Zement-Kartusche. Dabei wird eine Kombination aus einem gasdurchlässigen Sterilisationskolben und einem gasundurchlässigen Dichtungskolben verwendet. Das Durchmischen der Ausgangskomponenten in einem Mischraum erfolgt dadurch, dass Mischflügel, die an einem Mischstab befestigt sind, in dem Mischraum bewegt werden. Die Bewegung erfolgt durch manuelles Hineinstoßen, Herausziehen und Drehen des Mischstabs, wobei der Mischstab durch eine Durchführung nach außen aus dem Mischraum herausgeführt ist und in einem manuell bedienbaren Griffstück endet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

Bei den bisher üblichen Vakuummischsystemen wird die Vermischung durch manuell zu betätigende Mischvorrichtungen vorgenommen. Die Mischergebnisse sind nur bedingt reproduzierbar, weil individuelle Einflüsse der Anwender, wie unterschiedlicher Energieeintrag durch variable Mischintensität und Mischdauer, zum Tragen kommen. Das bedeutet, dass Schwankungen in den Zementeigenschaften auftreten können. Zudem entsteht beim Mischen des PMMA-Knochenzements immer auch ein Arbeitsaufwand, der im oft hektischen Alltag bei Operationen hinderlich sein kann. Ein unzureichendes Durchmischen des Knochenzements kann in einer Verschlechterung der Verbindung der zementierten Knochen und Prothesen resultieren, der absolut unerwünscht ist.

DE 10 2011 119 377 B3 offenbart eine Mischvorrichtung entsprechend dem Oberbegriff des Anspruchs 1 und ein Verfahren entsprechend dem Oberbegriff des Anspruchs 17.

Die Aufgabe der Erfindung besteht also darin, die Nachteile des Stands der Technik zu überwinden. Insbesondere soll eine Mischvorrichtung für Polymethylmethacrylat-Knochenzement bereitgestellt werden, bei der der Prozess der Vermischung des Zementpulvers mit der Monomerflüssigkeit reproduzierbar und unabhängig vom medizinischen Anwender erfolgen kann. Die Mischvorrichtung soll unabhängig von externen Energiequellen arbeiten. Weiterhin soll ein geschlossenes Vakuummischsystem entwickelt werden, in der die Mischvorrichtung integriert ist. Das Vakuummischsystem soll eine Zementkartusche enthalten, in der das Zementpulver gelagert ist, sowie einen davon separaten Vorratsbehälter, in dem sich die Monomerflüssigkeit befindet. Die Monomerflüssigkeit wird dadurch getrennt von dem Zementpulver gelagert. Vor und während der Vermischung der beiden Zementkomponenten, dem Zementpulver mit der Monomerflüssigkeit, soll ein Kontakt der medizinischen Anwender mit diesen Komponenten ausgeschlossen sein. Die Öffnung des Vorratsbehälters und der Transfer des Monomers sollen daher in einem geschlossenen System erfolgen. Das Zementpulver darf ebenfalls nicht in Kontakt zum medizinischen Anwender kommen. Zudem soll die Anwendung der Vorrichtung möglichst einfach sein und dennoch gleichzeitig einen gut durchmischten Knochenzementteig liefern.

Die Aufgaben der Erfindung werden gelöst durch eine Mischvorrichtung für Polymethylmethacrylat-Knochenzemente (PMMA-Knochenzemente) aufweisend einen Mischraum zum Mischen des PMMA-Knochenzements,
zumindest ein in dem Mischraum drehbar gelagertes Mischelement,
ein Getriebe, mit dem die Drehung des zumindest einen Mischelements anzutreiben ist,
mindestens ein elastisch verformbares Energiespeicherelement, das mit dem Getriebe verbunden ist, so dass mit einer elastischen Energie aus dem Energiespeicherelement das Getriebe anzutreiben ist und bei Abgabe von elastischer Energie aus dem Energiespeicherelement das zumindest eine Mischelement über das Getriebe in dem Mischraum drehbar ist. Dabei wird die Bewegung des zumindest einen Mischelements über eine Kurvenscheibe bei der Drehbewegung so beeinflusst, dass das zumindest eine Mischelement während der Drehbewegung auch eine periodische axiale Hubbewegung durchführt, wobei mit der Kurvenscheibe die axiale Bewegung der Rührwelle zu erzeugen ist, so dass die Rührwelle während der Drehbewegung auch eine periodische axiale Hubbewegung durchführt.

Es reicht erfindungsgemäß aus, wenn in dem elastisch verformbaren Energiespeicherelement die Energie für eine einzige Durchmischung der Ausgangskomponenten des PMMA-Knochenzements mit Hilfe des zumindest einen Mischelements enthalten ist.

Der PMMA-Knochenzement wird bevorzugt aus zwei Komponenten gemischt, wobei die erste Komponente ein Pulver oder eine Paste ist und die zweite Komponente fluid, bevorzugt flüssig ist, besonders bevorzugt ein Zementpulver und eine Monomerflüssigkeit ist.

Bei erfindungsgemäßen Mischvorrichtungen kann auch vorgesehen sein, dass das zumindest eine Mischelement auf einer drehbar gelagerten Rührwelle angeordnet ist und mit dem Getriebe eine Drehung der Rührwelle anzutreiben ist, wobei bevorzugt die Rührwelle durch eine gasdichte oder druckdichte Durchführung aus dem Mischraum herausgeführt ist und außerhalb des Mischraums mit dem Getriebe verbunden ist.

Die Rührwelle ist zur Übertragung der Energie von dem elastisch verformbaren Energiespeicherelement besonders gut geeignet, da hierdurch eine Drehbewegung besonders effizient zu übertragen ist. Zudem kann eine solche Rührwelle besonders gut gelagert und beim Durchgang in den Mischraum abgedichtet werden.

Es kann vorgesehen sein, dass das Getriebe formschlüssig oder kraftschlüssig mit der Rührwelle verbunden ist. Hierdurch wird ein stabiler und robuster Antrieb mit geringen Energieverlusten ermöglicht.

Bei Mischvorrichtungen mit Rührwelle kann auch vorgesehen sein, dass die Rührwelle hohl ist und eine Leitung für eine flüssige Ausgangskomponente des PMMA-Knochenzements bildet, bevorzugt eine Leitung für eine Monomerflüssigkeit als Ausgangskomponente des PMMA-Knochenzements bildet. Die Rührwelle ist dabei innen hohl.

Hierdurch kann erreicht werden, dass die flüssige Ausgangkomponente ins Innere des Mischraums eingeleitet werden kann. Insbesondere bei einer periodischen linearen axialen Bewegung der Rührwelle kann eine bessere Verteilung der flüssigen beziehungsweise fluiden Ausgangskomponente, insbesondere der Monomerflüssigkeit, in der anderen Ausgangskomponente, insbesondere dem Zementpulver, in dem Mischraum erreicht werden.

Mit einer Weiterentwicklung der vorliegenden Erfindung wird vorgeschlagen, dass das zumindest eine Mischelement eine Mehrzahl von Mischflügeln ist, wobei zumindest während der Drehung der Mischflügel im Mischraum sich die Mischflügel radial zur Drehachse in den Mischraum erstrecken und gegen eine Ebene senkrecht zur Drehachse geneigt sind.

Mischflügel sind zur Durchmischung des Innenraums besonders gut geeignet, da sie einerseits mit geringem Aufwand anzuwenden sind und bei geringem Widerstand beim Durchschneiden des PMMA-Knochenzementteigs eine gute Mischwirkung erzielen.

Dabei kann vorgesehen sein, dass die Mischflügel an der Rührwelle befestigt oder zu befestigen sind, wobei die Mischflügel axial zueinander versetzt auf der Rührwelle angeordnet sind.

Hierdurch wird eine gute Durchmischung des PMMA-Knochenzements auf der ganzen axialen Länge des Mischraums erreicht.

Dabei kann wiederum vorgesehen sein, dass die Mischflügel über ein Gelenk an der Rührwelle befestigt sind, so dass die Mischflügel sich an die Rührwelle anlegen lassen.

Hierdurch kann die Rührwelle mit den Mischflügeln daran leichter in den Mischraum eingeführt beziehungsweise herausgezogen werden.

Bei Mischvorrichtungen mit Mischflügeln kann auch vorgesehen sein, dass die Mischflügel lösbar an der Rührwelle befestigt sind, so dass die Mischflügel beim Herausziehen der Rührwelle aus dem Mischraum von der Rührwelle getrennt werden, insbesondere abgebrochen werden.

Die Mischflügel verbleiben dann im Mischraum. Hierdurch kann die Rührwelle bequem aus dem Mischraum entfernt beziehungsweise herausgezogen werden, ohne dass die Mischflügel das verhindern oder behindern. Insbesondere kann die Rührwelle auch aus der Durchführung für die Rührwelle in den Mischraum herausgezogen werden.

Mit einer Weiterbildung der erfindungsgemäßen Mischvorrichtung kann auch vorgesehen sein, dass das Getriebe und/oder das elastisch verformbare Energiespeicherelement durch zumindest eine lösbare mechanische Sicherung derart blockiert ist, dass eine Abgabe der Energie aus dem Energiespeicherelement verhindert ist, wobei bevorzugt die zumindest eine mechanische Sicherung eine Sperrklinke und/oder ein Sperrstift ist.

Hierdurch kann ein besonders einfaches und benutzerfreundliches Auslösen und Anwenden der Mischvorrichtung erreicht werden. Die mechanische Sicherung verhindert eine ungewollte Abgabe der elastischen Energie aus dem elastischen Energiespeicherelement. Nach Lösen der mechanischen Sicherung wird die elastische Energie aus dem Energiespeicherelement freigesetzt.

Gemäß einer bevorzugten Ausführungsform kann auch vorgesehen sein, dass das Getriebe ein Zahnradgetriebe oder ein Reibradgetriebe oder ein Zugmittelgetriebe ist.

Diese Getriebe sind besonders aufgrund ihres einfachen und kostengünstigen Aufbaus sowie ihrer Fehlerunanfälligkeit besonders gut zum Aufbau erfindungsgemäßer Mischvorrichtungen geeignet.

Des Weiteren kann vorgesehen sein, dass das elastisch verformbare Energiespeicherelement ein Federelement ist, das mit dem Getriebe verbunden ist, wobei das Getriebe durch die Federkraft des gespannten Federelements anzutreiben ist, wobei bevorzugt das Federelement ein gespanntes oder spannbares Federelement ist, besonders bevorzugt eine Metallfeder ist, ganz besonders bevorzugt eine Stahlschenkelfeder, eine Stahlblattfeder oder eine Stahlspiralfeder ist.

Mit solchen Federelementen ist ein besonders einfacher und kostengünstiger Aufbau realisierbar, da diese besonders gut zum Antreiben von Drehbewegungen geeignet sind. Es wurde im Rahmen der vorliegenden Erfindung gefunden, dass die in einem solchen Federelement gespeicherte elastische Energiemenge bei einer zur Größe der Mischvorrichtung beziehungsweise bei einer zur Größe eines damit aufgebauten Vakuummischsystems passenden Dimensionierung für eine ausreichende Durchmischung auch zäher PMMA-Knochenzemente geeignet ist.

Es kann ferner vorgesehen sein, dass eine Abgabe von elastischer Energie aus dem Energiespeicherelement eine Drehbewegung des Getriebes bewirkt, das Getriebe eine Übersetzung von wenigstens 2:1 auf das zumindest eine Mischelement bewirkt, bevorzugt mit einer Übersetzung von wenigstens 5:1, besonders bevorzugt von mindestens 8:1 auf das zumindest eine Mischelement bewirkt.

Eine Übersetzung von wenigstens 2:1 bedeutet dabei, dass bei einer Umdrehung des Getriebes zumindest zwei Umdrehungen des zumindest einen Mischelements im Mischraum erfolgt. Bevorzugt bewirkt das Getriebe eine Übersetzung von maximal 32:1 auf das zumindest eine Mischelement aus, besonders bevorzugt von maximal 20:1. Mit der Übersetzung wird eine starke Durchmischung des PMMA-Knochenzements erreicht und das für die Durchmischung des zähen PMMA-Knochenzementteigs notwendige hohe Drehmoment bereitgestellt.

Hierdurch wird eine Verbesserung der Mischwirkung durch die axiale Bewegung der Mischelemente im Mischraum erzielt.

Die der vorliegenden Erfindung zugrundeliegenden Aufgaben werden auch gelöst durch ein Vakuummischsystem zum Mischen eines PMMA-Knochenzements aufweisend eine erfindungsgemäße Mischvorrichtung, ferner aufweisend
den Mischraum, in dem ein Zementpulver enthalten ist,
einen Monomerbehälter, der mit einem fluiden oder flüssigen Monomer gefüllt ist und einem Leitungsmittel, das den Monomerbehälter mit dem Mischraum flüssigkeitsdurchlässig verbindet oder mit dem der Monomerbehälter mit dem Mischraum flüssigkeitsdurchlässig verbindbar ist.

Hierdurch wird gleich eine komplette Vorrichtung geschaffen, mit der eine PMMA-Knochenzement und dessen Ausgangskomponenten gelagert und gemischt werden können.

Bei solchen Vakuummischsystemen kann vorgesehen sein, dass das Vakuummischsystem einen Standfuß aufweist, an dem der Mischraum lösbar befestigt ist und an dem der Monomerbehälter befestigt ist, wobei das Leitungsmittel und das elastische Energiespeicherelement in oder an dem Standfuß angeordnet sind.

Hierdurch wird der Aufbau und die Anwendung des Vakuummischsystems vereinfacht und stabilisiert.

Ferner kann vorgesehen sein, dass sich eine Antriebsachse, insbesondere die Rührwelle, durch eine Dichtung aus dem Mischraum heraus erstreckt und an dem außerhalb des Mischraums angeordneten Teil der Antriebsachse, insbesondere der Rührwelle, ein Zahnrad oder Reibrad angebracht ist, das mit dem Getriebe verbunden ist, bevorzugt mit einem Zahnrad, Reibrad, Zahnriemen oder Reibriemen des Getriebes verbunden ist, wobei die Antriebsachse, insbesondere die Rührwelle, über das Zahnrad oder Reibrad anzutreiben ist.

Hierdurch wird ein besonders einfach und kostengünstig aufzubauendes Vakuummischsystem erzielt, das zuverlässig und störungsunanfällig arbeitet.

Des Weiteren kann vorgesehen sein, dass der Mischraum ein Teil einer Zementkartusche ist, wobei die Zementkartusche einseitig mit einem Austragskolben oder einem Austragskolbensystem verschlossen ist, wobei der Austragskolben oder das Austragskolbensystem in dem Mischraum axial beweglich und zum Austreiben des fertig gemischten PMMA-Knochenzements vorgesehen ist, der damit gebildete Verschluss für die Partikel des Zementpulvers undurchlässig ist und für Gas, insbesondere für Ethylenoxid, durchlässig ist, wobei die Zementkartusche vor der Vermischung der Zementkomponenten derart Vakuumdicht verschließbar ist, dass ein durch Vakuumeinwirkung bewirkter Transfer der Monomerflüssigkeit in den Mischraum der Zementkartusche zum Zementpulver möglich ist.

Hierdurch ist das Vakuummischsystem zusätzlich zum Sterilisieren und Austragen des fertig gemischten PMMA-Knochenzements sowie zum Mischen unter Vakuum geeignet.

Es kann auch vorgesehen sein, dass eine Vorrichtung zur Vakuumerzeugung in das Vakuummischsystem integriert ist.

Hierdurch wird das Vakuummischsystem weiter komplettiert und von einer externen Vakuumquelle unabhängig.

Die der vorliegenden Erfindung zugrundeliegenden Aufgaben werden auch gelöst durch ein Verfahren zum Mischen eines Zements, insbesondere eines PMMA-Knochenzements, bei dem zumindest zwei Ausgangskomponenten des Zements in einem Mischraum mit zumindest einem Mischelement gemischt werden, wobei das zumindest eine Mischelement in dem Mischraum um eine Drehachse gedreht wird und dabei die Ausgangskomponenten im Mischraum mischt, wobei die Drehung des zumindest einen Mischelements über ein Getriebe angetrieben wird und die Energie zum Antrieb des Getriebes von einem elastisch verformten Energiespeicherelement entnommen wird, wobei sich das elastisch verformte Energiespeicherelement bei der Energieentnahme entspannt, das Mischelement periodisch in axialer Richtung bewegt wird und dabei die Ausgangskomponenten im Mischraum mischt und die periodische axiale Bewegung des zumindest einen Mischelements über eine Kurvenscheibe und ein Getriebe angetrieben wird.

Bevorzugt kann dabei vorgesehen sein, dass der Mischraum beim Mischen der Ausgangskomponenten geschlossen ist, bevorzugt gasdicht und/oder druckdicht verschlossen ist.

Mit dem erfindungsgemäßen Verfahren wird ferner vorgeschlagen, dass das Verfahren mit einer erfindungsgemäßen Mischvorrichtung oder einem erfindungsgemäßen Vakuummischsystem durchgeführt wird.

Des Weiteren kann vorgesehen sein, dass eine erste Ausgangskomponente pulverförmig und bereits in dem Mischraum enthalten ist und eine zweite Ausgangskomponente eine Flüssigkeit ist, die in den Mischraum eingeleitet wird, bevorzugt mit einem Vakuum in den Mischraum eingesaugt wird.

Ferner kann vorgesehen sein, dass die Energie zum Antrieb des Getriebes und der Bewegung der Kurvenscheibe von dem elastisch verformten Energiespeicherelement entnommen wird, wobei sich das elastisch verformte Energiespeicherelement bei der Energieentnahme entspannt.

Es kann auch vorgesehen sein, dass nach dem Mischen des Zements eine Rührwelle, an der das zumindest eine Mischelement in Form von Mischflügeln befestigt ist, aus dem Mischraum herausgezogen wird, wobei die Mischflügel abbrechen und im Mischraum verbleiben oder die Mischflügel sich an die Rührwelle anlegen und mit der Rührwelle aus dem Mischraum herausgezogen werden.

Bevorzugt kann auch vorgesehen sein, dass das Energiespeicherelement eine gespannte Metallfeder ist, die sich bei der Abgabe von elastischer Energie an das Getriebe entspannt und dadurch das Getriebe angetrieben wird.

Bevorzugt rollt sich eine gespannte Spiralfeder aus Stahl als Energiespeicherelement zur Abgabe von elastischer Energie an das Getriebe ab.

Schließlich kann auch vorgesehen sein, dass die elastische Energie aus dem Energiespeicherelement entnommen wird, indem eine mechanische Sicherung gelöst wird, die das Getriebe und/oder das elastisch verformbare Energiespeicherelement blockiert, wobei nach dem Lösen die elastische Energie von dem Energiespeicherelement an das Getriebe abgegeben wird, wobei bevorzugt eine Sperrklinke und/oder ein Sperrstift als mechanische Sicherung verwendet wird, die besonders bevorzugt in das Getriebe eingreift und das Getriebe arretiert.

Der Erfindung liegt die überraschende Erkenntnis zugrunde, dass es durch die Entspannung eines elastisch verformten Energiespeicherelements, das Mischelemente über ein Getriebe antreibt, gelingt, eine Mischvorrichtung und ein Vakuummischsystem für PMMA-Knochenzemente sowie ein Verfahren zur Mischung eines Zements, insbesondere eines PMMA-Knochenzements, bereitzustellen, bei dem keine manuelle Durchmischung des PMMA-Knochenzements notwendig ist und in dem bereits die für die Mischung des PMMA-Knochenzements notwendige Energie gespeichert ist. Eine externe Energiequelle ist dadurch überflüssig. Die erfindungsgemäßen Vorrichtungen und das erfindungsgemäße Verfahren kommen dabei vollständig ohne elektrische Antriebe oder Motoren aus und sind auch für zähe PMMA-Knochenzemente gut geeignet. Durch die Standardisierung des Mischvorgangs können Unregelmäßigkeiten und Unterschiede in den physikalischen Eigenschaften des fertig gemischten PMMA-Knochenzements verringert oder sogar ausgeschlossen werden. Die Anwendung ist deutlich vereinfacht, was sich insbesondere bei oft hektischen Vorgängen während einer Operation (OP) positiv auswirkt. Der Aufbau bleibt dabei kostengünstig und das Produkt kann daher auch als Einmalartikel angeboten werden, was für die hygienischen Anforderungen im OP-Bereich sinnvoll ist. Mit erfindungsgemäßen Verfahren und Vorrichtungen kann ein PMMA-Knochenzement in gleichbleibender Qualität hergestellt werden. Das erfindungsgemäße Vakuummischsystem ist zudem zur Lagerung der Ausgangskomponenten und zur Applikation des fertig gemischten Knochenzements geeignet.

Erfindungsgemäße Mischvorrichtungen für PMMA-Knochenzemente können beispielsweise aufweisen:
a) mindestens eine drehbar gelagerte Rührwelle, die mit mindestens einem Mischelement verbunden ist,
b) ein Getriebe, das formschlüssig oder kraftschlüssig mit der Rührwelle verbunden ist,
c) mindestens ein elastisch deformierbares Energiespeicherelement, das mit dem Getriebe verbunden ist,
   wobei bei Entspannung des Energiespeicherelements eine Drehbewegung des Getriebes bewirkt wird, wodurch das Getriebe die Drehbewegung auf die Rührwelle mit dem verbundenen Mischelement überträgt.

Als elastisch deformierbares Energiespeicherelement werden Metallfedern bevorzugt, wobei ganz besonders Stahlschenkelfedern, Stahlblattfedern und Stahlspiralfedern bevorzugt sind. Daneben können aber auch Stäbe, Spiralfedern aus Elastomeren verwendet werden.

Die Federn können durch Deformation gespannt werden. Dadurch wird mechanische Energie gespeichert. Im gespannten Zustand wird das Energiespeicherelement durch mindestens eine lösbare, mechanische Sicherung des Getriebes so blockiert, dass eine spontane, unbeabsichtigte Abgabe der Energie des Energiespeicherelements verhindert wird, wobei diese Sicherung bevorzugt als Sperrklinke und/oder als Sperrstift ausgebildet ist. Die Sicherung kann von außen direkt durch den Anwender gelöst werden. Gemäß einer erfindungsgemäß besonders bevorzugten Ausgestaltung kann die Sicherung mit der Auslösung von anderen Funktionen, wie zum Beispiel dem Öffnen des Monomerbehälters, gekoppelt werden.

Für die Energieübertragung von dem Energiespeicherelement zur Rührwelle beziehungsweise zu dem zumindest einen Mischelement ist es erforderlich, ein Getriebe dazwischen zu schalten, um eine ausreichende Anzahl von Drehbewegungen auf die Rührwelle übertragen zu können. Dabei wird ein Zahnradgetriebe oder ein Reibradgetriebe oder ein Zugmittelgetriebe bevorzugt, das mindestens zwei Drehbewegungen, bevorzugt mindestens fünf und ganz besonders bevorzugt mindestens acht Umdrehungen der Rührwelle beziehungsweise des zumindest einen Mischelements bei der durch Entspannung des Energiespeicherelements verursachten Bewegung des Getriebes durch Übersetzung erzeugt. Bei dem Zugmittelgetriebe können Zahnriemen und/oder Reibriemen zur Übertragung der Drehmomente beziehungsweise der Drehbewegung verwendet werden. Alternativ kann auch ein Getriebe unter Nutzung von Zahnstangen verwendet werden.

Es ist vorteilhaft, wenn die Rührwelle mit der mindestens einen Kurvenscheibe bei der Drehbewegung wechselwirkt, wodurch die Rührwelle während der Drehbewegung eine periodisch axiale Hubbewegung durchführt. Dadurch ist es möglich, dass das mindestens eine Mischelement bei seiner Drehung auf und ab in der Kartusche bewegt wird. Dadurch werden bei geeigneter Auslegung der Rührwerkslänge und der Kurvenscheibe bei der Rührbewegung alle Bereiche der Innenseite des Mischraums erfasst. Es verbleiben dadurch keine vom Mischelement nicht erfassten, ungemischten Bereiche des Zementpulvers und der Monomerflüssigkeit im Mischraum. Es ist dabei vorteilhaft, wenn das Mischelement sich elastisch an die Innenwand des Mischraums der Zementkartusche anpressende Abstreifelemente enthält.

Ein bevorzugtes Ausführungsbeispiel ist auch ein Vakuummischsystem mit einer erfindungsgemäßen Mischvorrichtung, das einen Mischraum, in dem Zementpulver gelagert ist, einen separaten Monomerbehälter, ein Leitungsmittel, das den Monomerbehälter mit dem Mischraum flüssigkeitsdurchlässig verbindet, und ein Fußteil, das mit dem Mischraum und dem Monomerbehälter verbunden ist, aufweist. Das Vakuummischsystem umfasst beispielsweise
a) mindestens eine drehbare Rührwelle, wobei ein erster Abschnitt der Rührwelle in dem Mischraum angeordnet ist, der mit mindestens einem Mischelement verbunden ist, und ein zweiter Abschnitt der Rührwelle außerhalb des Mischraums in einem separaten Fußteil angeordnet ist, wobei dieser Abschnitt an seinem Ende als Zahnrad oder Reibrad ausgebildet ist und um seine Längsachse drehbar gelagert ist,
b) einem Zahnradgetriebe oder Reibradgetriebe oder Zugmittelgetriebe, das in Eingriff mit dem als Zahnrad oder Reibrad ausgebildeten Ende der Rührwelle steht, und
c) mindestens einer Feder, die auf das Zahnradgetriebe oder Reibradgetriebe oder Zugmittelgetriebe einwirkt.

Das erfindungsgemäße Vakuummischsystem kann auch dadurch charakterisiert sein, dass das zumindest eine Mischelement mit der Rührwelle reversibel verbunden ist und das beim Herausziehen der Rührwelle durch Abstreifen von der Rührwelle gelöst werden kann.

Bei einer anderen Ausgestaltung des Vakuummischsystems kann das mindestens eine Mischelement beim Herausziehen der Rührwelle aus dem Mischraum an die Rührwelle angeklappt werden. Dadurch ist es möglich, die Rührwelle aus dem Mischraum nach erfolgter Vermischung der Zementkomponenten zu entfernen.

Das erfindungsgemäße Vakuummischsystem kann ferner dadurch charakterisiert sein, dass die Zementkartusche mit einem Kolben oder Kolbensystem verschlossen ist, der oder das undurchlässig für die Partikel des Zementpulvers ist, jedoch für Ethylenoxid während der Sterilisation durchlässig ist und das vor der Vermischung der Zementkomponenten so Vakuum-dicht verschlossen werden kann, dass ein durch Vakuumeinwirkung bewirkter Transfer der Monomerflüssigkeit in die Mischkartusche zum Zementpulver möglich ist.

Besonders vorteilhaft ist es, wenn eine Vorrichtung zur Vakuumerzeugung in das Vakuummischsystem integriert ist. Diese Vorrichtung zur Vakuumerzeugung kann zum Beispiel selbst durch eine Feder oder ein Federsystem angetrieben werden. Daneben ist es auch möglich, dass die Vakuumerzeugung durch eine integrierte Druckgaspatrone erfolgt, wobei zur Unterdruckerzeugung eine Venturi-Düse genutzt wird.

Im Folgenden werden weitere Ausführungsbeispiele der Erfindung anhand von fünf schematisch dargestellten Abbildungen erläutert, ohne jedoch dabei die Erfindung zu beschränken. Dabei zeigt:
Abbildung 1: eine schematische Querschnittansicht eines Ausschnitts eines erfindungsgemäßen Vakuummischsystems mit einer erfindungsgemäßen Mischvorrichtung zum Umsetzen eines erfindungsgemäßen Verfahrens;
Abbildung 2: eine schematische Querschnittansicht des Aufbaus nach Abbildung 1 entsichert und mit laufendem Antrieb;
Abbildung 3: eine perspektivische schematische Teilschnittansicht des Aufbaus nach Abbildung 1;
Abbildung 4: eine perspektivische schematische Teilschnittansicht eines Ausschnitts eines alternativen erfindungsgemäßen Vakuummischsystems mit einer erfindungsgemäßen Mischvorrichtung zum Umsetzen eines erfindungsgemäßen Verfahrens; und
Abbildung 5: eine schematische Querschnittansicht des Aufbaus nach Abbildung 4.

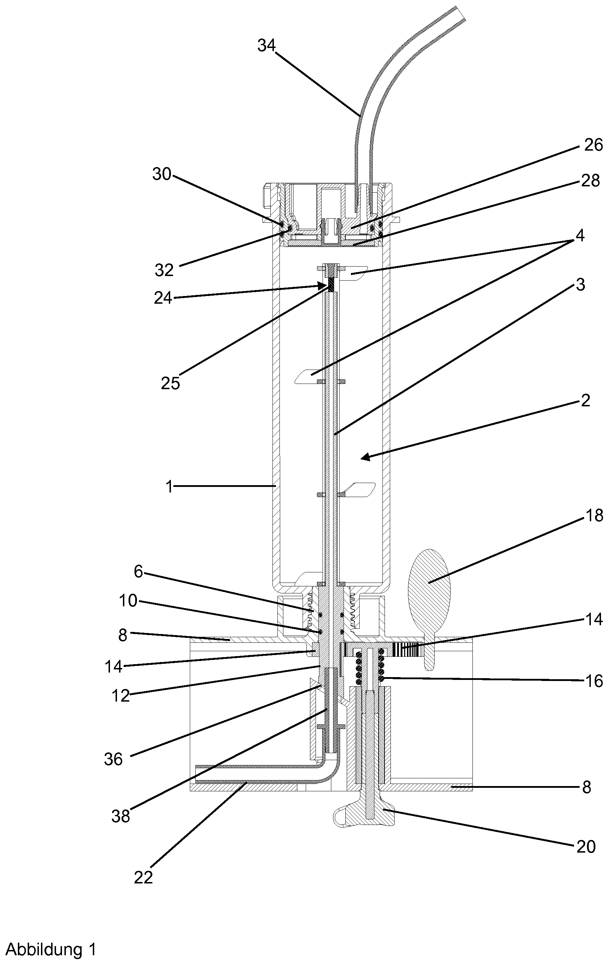

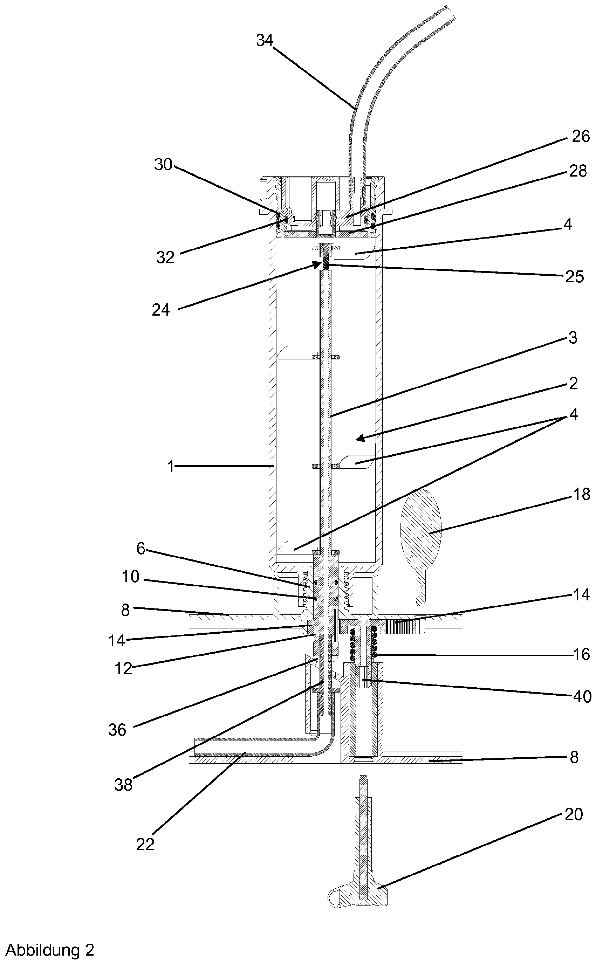

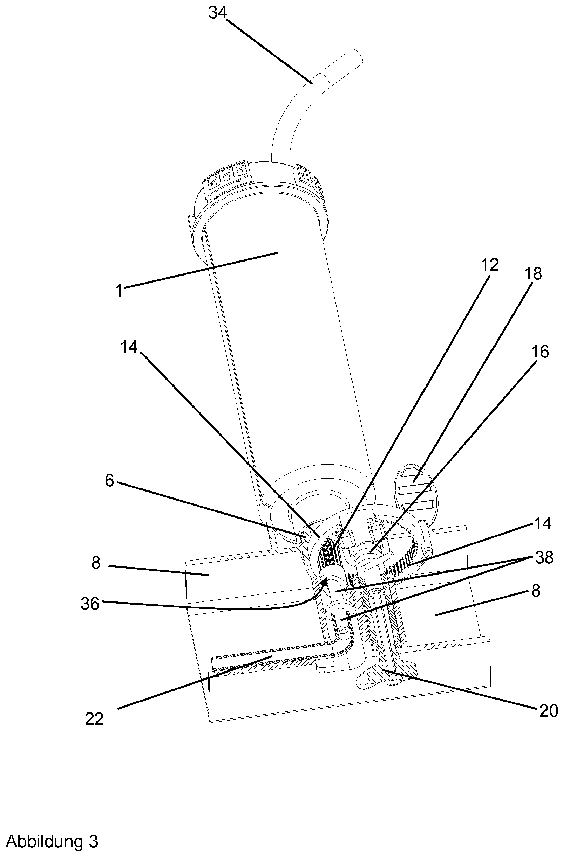

Die Abbildungen 1 und 2 zeigen schematische Querschnittansichten eines Ausschnitts eines erfindungsgemäßen Vakuummischsystems mit einer erfindungsgemäßen Mischvorrichtung zum Umsetzen eines erfindungsgemäßen Verfahrens und Abbildung 3 eine perspektivische schematische Teilschnittansicht hiervon. In den Abbildungen 1 und 3 ist ein arretierter Zustand und in Abbildung 2 ein entarretierter Zustand dargestellt.

Das Vakuummischsystem hat eine Zementkartusche 1, die aus einem Kunststoff besteht und die einen zylindrischen Mischraum 2 in ihrem Inneren begrenzt. Durch eine bodenseitige Austragsöffnung (in den Abbildungen 1 und 2 unten) ist eine drehbar gelagerte Rührwelle 3 geführt, an der vier Mischflügel 4 als Mischelemente angebracht sind. Die Mischflügel 4 sind über Gelenke mit der Rührwelle 3 verbunden und können so an die Rührwelle 3 angelegt werden, um die Rührwelle 3 durch die bodenseitige Austragsöffnung in den Mischraum 2 einführen und herausziehen zu können. Es können alternativ auch Gruppen von Mischflügeln 4 als Mischelemente an der Rührwelle 3 angebracht werden.

Die Austragsöffnung weist ein Innengewinde auf und die Zementkartusche 1 ist mit diesem Innengewinde auf ein Außengewinde eines Stutzens 6 geschraubt. Der Stutzen 6 ist Teil eines Standfußes 8 auf dem das gesamte Vakuummischsystem aufgebaut ist. Die Rührwelle 3 dichtet die Austragsöffnung ab. Dazu sind an der Rührwelle 3 zwei Dichtungsringe 10 aus Gummi am Außenumfang der Rührwelle 3 angeordnet. Die Rührwelle 3 ist in diesem Bereich verbreitert, um das Einschieben und Herausziehen der Rührwelle 3 mit angelegten Mischflügeln 4 zu ermöglichen. Im Bereich der Mischflügel 4 ist die Rührwelle 3 dementsprechend mit einem kleineren Außendurchmesser versehen.

Unterhalb der Dichtungen 10 (in den Abbildungen 1 und 2 unten) ist in der Rührwelle 3 ein Zahnrad 12 ausgebildet, mit dem die Rührwelle 3 um die eigene Drehachse gedreht werden kann, beziehungsweise mit dem eine Drehbewegung der Rührwelle 3 um die eigene Achse angetrieben werden kann. Bei einer solchen Drehung drehen sich auch die Mischflügel 4 in dem Mischraum 2 und sorgen so für eine Durchmischung eines im Mischraum 2 enthaltenen PMMA-Knochenzements (nicht gezeigt) beziehungsweise seiner Ausgangskomponenten (nicht gezeigt). Die Mischflügel 4 können sich durch die dabei auftretenden Fliehkräfte von der Rührwelle 3 abheben. An den Mischflügeln 4 sind Abstreifer (nicht gezeigt) vorgesehen, die an der Innenwand der Zementkartusche 1 beziehungsweise an den Begrenzungen des Mischraums 2 im Betrieb, das heißt während der Drehung, anliegen, so dass eine vollständige Durchmischung des Mischguts im Mischraum 2 erfolgen kann.

In das Zahnrad 12 der Rührwelle 3 greift ein Zahnrad 14 eines Getriebes. Das Zahnrad 14 weist eine innenliegende Zahnung auf und hat zumindest doppelt so viele Zähne wie das Zahnrad 12. Hierdurch wird eine Übersetzung erreicht, so dass bei einer Dreivierteldrehung des Zahnrads 14 sich das Zahnrad 12 und damit die Rührwelle 3 mehr als einmal vollständig um die eigene Achse dreht.

Um die Bewegung des Zahnrads 14 des Getriebes anzutreiben, ist an dem Zahnrad 14 eine gespannte Spiralfeder 16 aus Metall befestigt. Das Getriebe und die Feder 16 sind innerhalb des Standfußes 8 angeordnet, der hierzu ein Gehäuse aus Kunststoff bildet. Das Getriebe beziehungsweise das Zahnrad 14 ist im gesicherten Zustand (Abbildungen 1 und 3) mit einer Sicherung 18 in Form eines Stifts mit einem Griffstück gesichert. Die Sicherung 18 ist dazu durch eine Öffnung im Standfuß 8 hindurchgeführt und in eine Ausnehmung am äußeren Umfang des Zahnrads 14 gesteckt, so dass eine Bewegung des Zahnrads 14 blockiert ist und damit die gespannte Feder 16 sich nicht entspannen kann. Im entsicherten Zustand (Abbildung 2) ist die Sicherung 18 einfach herausgezogen worden. Die Spiralfeder 16 entspannt sich und treibt damit das Zahnrad 14 und damit die Rührwelle 3 an. Die in der gespannten Spiralfeder 16 gespeicherte elastische Energie kann so dazu genutzt werden, den Inhalt des Mischraums 2 mit Hilfe der Mischelemente 4 zu durchmischen. Die Spiralfeder 16 stellt somit ein elastisch verformbares Energiespeicherelement 16 dar, in dem elastische Energie als Kurzzeitantrieb für den Mischvorgang gespeichert werden kann.

Die Spiralfeder 16 kann mit einem Aufziehflügelstift 20 wie eine Uhr aufgezogen werden. Die Flügel des Aufziehflügelstifts 20 erleichtern dabei die Bedienbarkeit. Der Aufziehflügelstift 20 endet in einem Sechskant oder einer anderen von der zylindrischen Geometrie abweichenden Form. Der Sechskant kann dazu in ein Sechskantloch 40 (nur in Abbildung 2 zu sehen) gesteckt werden, das axial fest mit der Spiralfeder 16 verbunden ist. Es ist aber auch möglich das Vakuummischsystem beziehungsweise die Mischvorrichtung im vorgespannten Zustand ohne den Aufziehflügelstift 20 auszuliefern.

Im Standfuß 8 ist eine Leitung 22 angeordnet, die durchgängig mit einer Leitung in der Rührwelle 3 verbunden ist. Die Leitung 22 endet nicht, wie in den Abbildungen 1 bis 3 dargestellt, sondern setzt sich weiter fort (in den Abbildungen 1 bis 3 nach links) und ist dort mit einem Monomerflüssigkeitsbehälter (nicht gezeigt) verbunden. Die Rührwelle 3 ist also als ein Rohr ausgebildet. Das Rohr der Rührwelle 3 mündet über Öffnungen 24 und einen porösen Kern 25 in den Mischraum 2. Der poröse Kern 25 ist nicht für Pulver durchlässig aber für Flüssigkeiten durchlässig. Hierdurch kann sichergestellt werden, dass ein in dem Mischraum 2 enthaltenes Zementpulver (nicht gezeigt) als erste Ausgangskomponente für den PMMA-Knochenzement nicht in das Rohr der Rührwelle 3 eindringen kann, die Monomerflüssigkeit (nicht gezeigt) aus dem Monomerflüssigkeitsbehälter als zweite Ausgangskomponente für den PMMA-Knochenzement aber durch den porösen Kern 25 und die Öffnungen 24 über das Rohr der Rührwelle 3 und die Leitung 22 in den Mischraum 2 eingeleitet werden kann.

Auf der der Austragsöffnung gegenüberliegenden Seite (in den Abbildungen 1 und 2 oben) der Zementkartusche 1 ist in der Zementkartusche 1 ein zweiteiliger Austragskolben aus einem Dichtungskolben 26 und einem Sterilisationskolben 28 angeordnet. Der Austragskolben ist in dem Mischraum 2 axial beweglich angeordnet und am oberen Ende lösbar arretiert. Mit dem Austragskolben kann der Inhalt der Mischraums 2 bei herausgenommener Rührwelle 3 durch die Austragsöffnung herausgepresst werden. Der Sterilisationskolben 28 ist mit zwei umlaufenden Dichtungen 30 gegen die Innenwand der Zementkartusche 1 abgedichtet. Der Sterilisationskolben 28 umfasst eine Porenscheibe, durch die ein Pulver aus dem Mischraum 2 nicht nach außen dringen kann, ein sterilisierendes Gas, wie beispielsweise Ethylenoxid, aber eingeleitet werden kann. Nach dem der Mischraum 2 mit dem Zementpulver darin mit dem Ethylenoxid sterilisiert wurde, wird der Dichtungskolben 26 in den Sterilisationskolben 28 eingesetzt. Der Dichtungskolben 26 weist eine umlaufende Dichtung 32 auf, die den Dichtungskolben 26 gegen den Sterilisationskolben 28 abdichtet. Ineinander gesteckt bilden der Dichtungskolben 26 und der Sterilisationskolben 28 dann den zweiteiligen Austragskolben.

In dem Dichtungskolben 26 ist eine Durchführung mit einem Anschlussstutzen für einen Vakuumschlauch 34 angeordnet. Durch den Vakuumschlauch 34 und die Durchführung des Dichtungskolbens 26, kann der Mischraum 2 evakuiert werden. Dadurch kann zudem die Monomerflüssigkeit durch die Leitung 22 in den Mischraum 2 gesaugt werden und die beiden Ausgangskomponenten können im Mischraum 2 unter Vakuum gemischt werden. Dazu ist der Vakuumschlauch 34 an eine externe oder eine interne (zum Vakuummischsystem gehörende) Vakuumquelle angeschlossen.

Die Rührwelle 3 endet außerhalb des Mischraums 2 (in den Abbildungen 1 bis 3 unten) in einer Kurvenscheibe 36. Die Kurvenscheibe 36 ist durch eine Abschrägung des Endes der Rührwelle 3 gebildet. Die Kurvenscheibe 36 liegt auf einem ebenso abgeschrägten und feststehenden Stutzen des Standfußes 8 auf. Bei einer Drehung der Rührwelle 3 wird die Kurvenscheibe 36 bei einer vollen Umdrehung nach oben gedrückt und wieder abgesenkt. Dadurch wird bei einer Drehung der Rührwelle 3 diese mit den Mischelementen 4 periodisch im Mischraum 2 auf und ab bewegt. Hierdurch wird eine zusätzliche Durchmischung des Inhalts des Mischraums 2 erreicht. Damit sich das Rohr der Rührwelle 3 bei dieser Bewegung nicht von der Leitung 22 löst, ist ein Anschlussstück 38 in Form eines in Längsrichtung verschiebbaren Rohres vorgesehen, das in einem Anschluss der Leitung 22 verschiebbar ist.

Der PMMA-Knochenzement kann mit dem in den Abbildungen 1 bis 3 gezeigten Aufbau wie folgt hergestellt und gegebenenfalls appliziert werden:
Die Feder 16 ist gespannt und mit der Sicherung 18 arretiert oder die Feder 16 wird mit dem Aufziehflügelstift 20 gespannt und anschließend arretiert. Der Monomerflüssigkeitsbehälter wird geöffnet. Über den Vakuumschlauch 34 wird der Mischraum 2 mit dem Zementpulver darin evakuiert und die Monomerflüssigkeit durch die Leitung 22, das Anschlussstück 38, das Rohr der Rührwelle 3, den porösen Kern 25 und die Öffnungen 24 in den Mischraum 2 eingesaugt.

Die Sicherung 18 wird herausgezogen beziehungsweise entfernt. Die Feder 16 treibt das Zahnrad 14 an und das Zahnrad 14 treibt das Zahnrad 12 an. Dadurch wird die Rührwelle 3 gedreht und bewegt sich bei der Drehung durch das Gleiten der Kurvenscheibe 36 auf dem abgeschrägten Stutzen im Gehäuse des Standfußes 8 auf und ab. Dabei drehen, heben und senken sich auch die Mischflügel 4 im Mischraum 2, wodurch die Ausgangskomponenten im Mischraum 2 durchmischt werden.

Nach erfolgter Durchmischung wird die Zementkartusche 1 mit dem gemischten Zementteig darin vom Standfuß 8 abgeschraubt und die Rührwelle 3 mit den Mischflügeln 4 daran aus der Austragsöffnung herausgezogen. In die Austragsöffnung wird ein Austragsrohr (nicht gezeigt) eingeschraubt, in dem ein zusätzlicher statischer Mischer enthalten sein kann, aber nicht enthalten sein muss. Die Arretierung des Austragskolbens mit der Innenwand der Zementkartusche 1 wird gelöst und der Austragskolben in Richtung der Austragsöffnung nach vorne getrieben. Dadurch wird der Inhalt des Mischraums 2 durch die Austragsöffnung ausgetrieben und der gemischte PMMA-Knochenzement kann so appliziert werden.

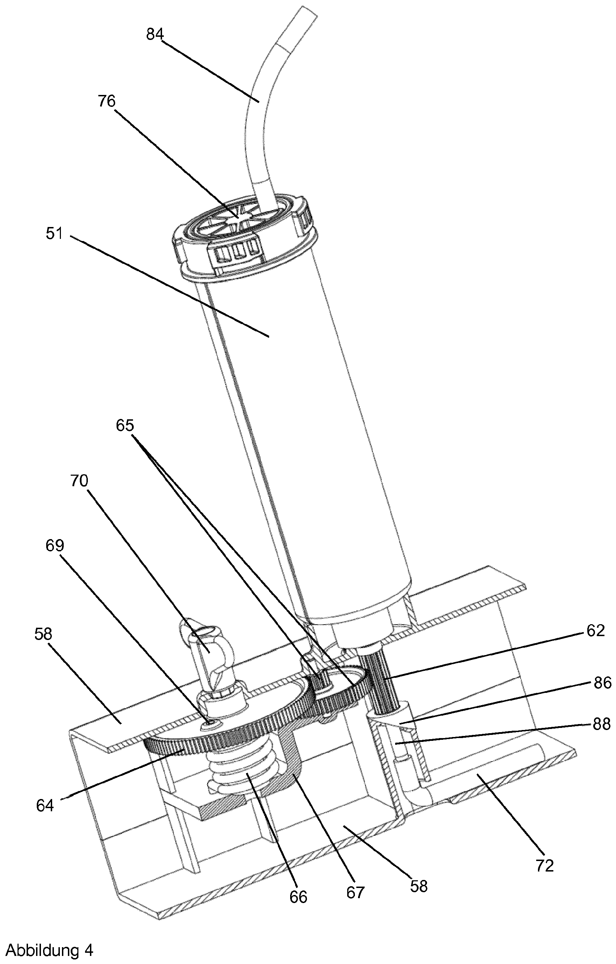

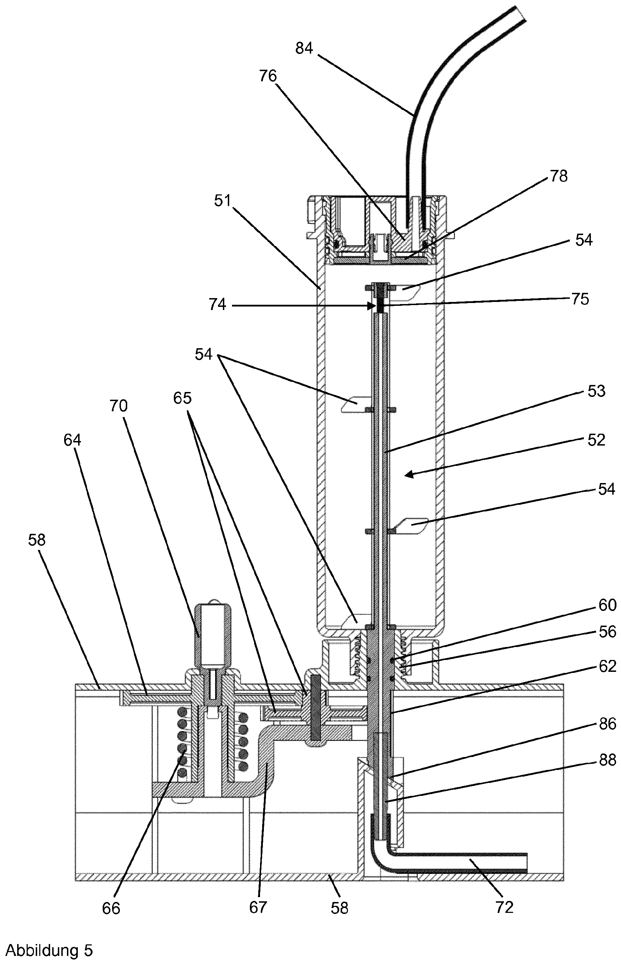

Die Abbildungen 4 und 5 zeigen eine perspektivische schematische Teilschnittansicht und eine schematische Querschnittansicht eines Ausschnitts eines alternativen erfindungsgemäßen Vakuummischsystems mit einer erfindungsgemäßen Mischvorrichtung zum Umsetzen eines erfindungsgemäßen Verfahrens.

Das Vakuummischsystem hat eine Zementkartusche 51, die aus einem Kunststoff besteht und die einen zylindrischen Mischraum 52 in ihrem Inneren begrenzt. Durch eine bodenseitige Austragsöffnung (in Abbildung 5 unten) ist eine drehbar gelagerte Rührwelle 53 geführt, an der vier Mischflügel 54 als Mischelemente angebracht sind. Die Mischflügel 54 sind über Gelenke mit der Rührwelle 53 verbunden und können so an die Rührwelle 53 angelegt werden, um die Rührwelle 53 durch die bodenseitige Austragsöffnung in den Mischraum 52 einführen und herausziehen zu können. Es können alternativ auch Gruppen von Mischflügeln 54 als Mischelemente an der Rührwelle 53 angebracht werden.

Die Austragsöffnung weist ein Innengewinde auf und die Zementkartusche 51 ist mit diesem Innengewinde auf ein Außengewinde eines Stutzens 56 geschraubt. Der Stutzen 56 ist Teil eines Standfußes 58 auf dem das gesamte Vakuummischsystem aufgebaut ist. Die Rührwelle 53 dichtet die Austragsöffnung ab. Dazu sind an der Rührwelle 53 zwei Dichtungsringe 60 aus Gummi am Außenumfang der Rührwelle 53 angeordnet. Die Rührwelle 53 ist in diesem Bereich verbreitert, um das Einschieben und Herausziehen der Rührwelle 53 mit angelegten Mischflügeln 54 zu ermöglichen. Im Bereich der Mischflügel 54 ist die Rührwelle 53 dementsprechend mit einem kleineren Außendurchmesser versehen.

Unterhalb der Dichtungen 60 (in den Abbildungen 4 und 5 unten) ist in der Rührwelle 53 ein Zahnrad 62 ausgebildet, mit dem die Rührwelle 53 um die eigene Drehachse gedreht werden kann, beziehungsweise mit dem eine Drehbewegung der Rührwelle 53 um die eigene Achse angetrieben werden kann. Bei einer solchen Drehung drehen sich auch die Mischflügel 54 in dem Mischraum 52 und sorgen so für eine Durchmischung eines im Mischraum 52 enthaltenen PMMA-Knochenzements (nicht gezeigt) beziehungsweise seiner Ausgangskomponenten (nicht gezeigt). Die Mischflügel 54 können sich durch die dabei auftretenden Fliehkräfte von der Rührwelle 53 abheben. An den Mischflügeln 54 sind Abstreifer (nicht gezeigt) vorgesehen, die an der Innenwand der Zementkartusche 51 beziehungsweise an den Begrenzungen des Mischraums 52 im Betrieb, das heißt während der Drehung, anliegen, so dass eine vollständige Durchmischung des Mischguts im Mischraum 52 erfolgen kann.

Das Zahnrad 62 der Rührwelle 53 wird von einem Zahnrad 64 über zwei weitere Zahnräder 65, die gemeinsam auf einer Achse angeordnet sind, angetrieben. Die Zahnräder 64, 65 bilden ein Getriebe mit mehrfacher Übersetzung zum Antrieb der Rührwelle 53. Das Zahnrad 64 weist im Unterschied zu der Ausführung nach den Abbildungen 1 bis 3 eine außenliegende Zahnung auf. Zudem weist das Getriebe nach dieser Ausführung mit den Zahnrädern 65 eine weitere zusätzliche Getriebestufe auf. Hierdurch wird eine hohe Übersetzung des Getriebes von wenigstens acht zu eins (8:1) erreicht, so dass bei einer vollständigen Drehung des Zahnrads 64 sich das Zahnrad 62 und damit die Rührwelle 53 zumindest achtmal vollständig um die eigene Achse dreht.

Um die Bewegung der Zahnräder 64, 65 des Getriebes anzutreiben, ist an dem Zahnrad 64 eine gespannte Spiralfeder 66 aus Metall oder Kunststoff befestigt. Das Getriebe und die Feder 66 sind innerhalb des Standfußes 58 angeordnet, der hierzu ein Gehäuse aus Kunststoff bildet. Das Getriebe beziehungsweise die Zahnräder 64, 65 sind im gesicherten Zustand mit einer Sicherung (nicht gezeigt) in Form eines Stifts mit einem Griffstück gesichert. Die Sicherung ist dazu durch eine Öffnung im Standfuß 58 hindurchgeführt und in eine Ausnehmung 69 im Zahnrad 64 gesteckt, so dass eine Bewegung des Zahnrads 64 blockiert ist und sich dadurch die gespannte Feder 66 nicht entspannen kann. Im entsicherten Zustand ist die Sicherung einfach herausgezogen. Die Spiralfeder 66 entspannt sich und treibt damit das Zahnrad 64 an, und darüber die Zahnräder 65 und damit die Rührwelle 63 an. Die in der gespannten Spiralfeder 66 gespeicherte elastische Energie kann so dazu genutzt werden, den Inhalt des Mischraums 52 mit Hilfe der Mischelemente 54 zu durchmischen. Die Spiralfeder 66 stellt somit ein elastisch verformbares Energiespeicherelement 66 dar, in dem elastische Energie als Kurzzeitantrieb für den Mischvorgang gespeichert werden kann.

Die Spiralfeder 66 kann mit einem Aufziehflügelstift 70 wie eine Uhr aufgezogen werden. Die Flügel des Aufziehflügelstifts 70 erleichtern dabei die Bedienbarkeit. Der Aufziehflügelstift 70 endet in einer flachen Platte oder einer anderen von der zylindrischen Geometrie abweichenden Form. Die Platte kann dazu in ein passendes Loch (nicht zu sehen) gesteckt werden, das axial fest mit der Spiralfeder 66 verbunden ist. Es ist aber auch möglich das Vakuummischsystem beziehungsweise die Mischvorrichtung im vorgespannten Zustand ohne den Aufziehflügelstift 70 auszuliefern.

Im Standfuß 58 ist eine Leitung 72 angeordnet, die durchgängig mit einer Leitung in der Rührwelle 53 verbunden ist. Die Leitung 72 endet nicht, wie in den Abbildungen 4 und 5 dargestellt, sondern setzt sich weiter fort (in den Abbildungen 4 und 5 nach rechts) und ist dort mit einem Monomerflüssigkeitsbehälter (nicht gezeigt) verbunden. Die Rührwelle 53 ist also als ein Rohr ausgebildet. Das Rohr der Rührwelle 53 mündet über Öffnungen 74 und einen porösen Kern 75 in den Mischraum 52. Der poröse Kern 75 ist nicht für Pulver durchlässig aber für Flüssigkeiten durchlässig. Hierdurch kann sichergestellt werden, dass ein in dem Mischraum 52 enthaltenes Zementpulver (nicht gezeigt) als erste Ausgangskomponente für den PMMA-Knochenzement nicht in das Rohr der Rührwelle 53 eindringen kann, die Monomerflüssigkeit (nicht gezeigt) aus dem Monomerflüssigkeitsbehälter als zweite Ausgangskomponente für den PMMA-Knochenzement aber durch den porösen Kern 75 und die Öffnungen 74 über das Rohr der Rührwelle 53 und die Leitung 72 in den Mischraum 52 eingeleitet werden kann.

Auf der der Austragsöffnung gegenüberliegenden Seite (in den Abbildungen 4 und 5 oben) der Zementkartusche 51 ist in der Zementkartusche 51 ein zweiteiliger Austragskolben aus einem Dichtungskolben 76 und einem Sterilisationskolben 78 angeordnet. Der Austragskolben ist in dem Mischraum 52 axial beweglich angeordnet und am oberen Ende lösbar arretiert. Mit dem Austragskolben kann der Inhalt der Mischraums 52 bei herausgenommener Rührwelle 53 durch die Austragsöffnung herausgepresst werden. Der Sterilisationskolben 78 ist mit zwei umlaufenden Dichtungen gegen die Innenwand der Zementkartusche 51 abgedichtet. Der Sterilisationskolben 78 umfasst eine Porenscheibe, durch die ein Pulver aus dem Mischraum 52 nicht nach außen dringen kann, ein sterilisierendes Gas, wie beispielsweise Ethylenoxid, aber eingeleitet werden kann. Nach dem der Mischraum 52 mit dem Zementpulver darin mit dem Ethylenoxid sterilisiert wurde, wird der Dichtungskolben 76 in den Sterilisationskolben 78 eingesetzt. Der Dichtungskolben 76 weist eine umlaufende Dichtung auf, die den Dichtungskolben 76 gegen den Sterilisationskolben 78 abdichtet. Ineinander gesteckt bilden der Dichtungskolben 76 und der Sterilisationskolben 78 dann den zweiteiligen Austragskolben.

In dem Dichtungskolben 76 ist eine Durchführung mit einem Anschlussstutzen für einen Vakuumschlauch 84 angeordnet. Durch den Vakuumschlauch 84 und die Durchführung des Dichtungskolbens 76, kann der Mischraum 52 evakuiert werden. Dadurch kann zudem die Monomerflüssigkeit durch die Leitung 72 in den Mischraum 52 gesaugt werden und die beiden Ausgangskomponenten können im Mischraum 52 unter Vakuum gemischt werden. Dazu ist der Vakuumschlauch 84 an eine externe oder eine interne (zum Vakuummischsystem gehörende) Vakuumquelle angeschlossen.

Die Rührwelle 53 endet außerhalb des Mischraums 52 (in den Abbildungen 4 und 5 unten) in einer Kurvenscheibe 86. Die Kurvenscheibe 86 ist durch eine Abschrägung des Endes der Rührwelle 53 gebildet. Die Kurvenscheibe 86 liegt auf einem ebenso abgeschrägten und feststehenden Stutzen des Standfußes 58 auf. Bei einer Drehung der Rührwelle 53 wird die Kurvenscheibe 86 bei einer vollen Umdrehung nach oben gedrückt und wieder abgesenkt. Dadurch wird bei einer Drehung der Rührwelle 53 diese mit den Mischelementen 54 periodisch im Mischraum 52 auf und ab bewegt. Hierdurch wird eine zusätzliche Durchmischung des Inhalts des Mischraums 52 erreicht. Damit sich das Rohr der Rührwelle 53 bei dieser Bewegung nicht von der Leitung 72 löst, ist ein Anschlussstück 88 in Form eines in Längsrichtung verschiebbaren Rohres vorgesehen, das in einem Anschluss der Leitung 72 verschiebbar ist.

Der PMMA-Knochenzement kann mit dem in den Abbildungen 4 und 5 gezeigten Aufbau wie folgt hergestellt und gegebenenfalls appliziert werden:
Die Feder 66 ist gespannt und mit der Sicherung arretiert oder die Feder 66 wird mit dem Aufziehflügelstift 70 gespannt und anschließend arretiert. Der Monomerflüssigkeitsbehälter wird geöffnet. Über den Vakuumschlauch 84 wird der Mischraum 52 mit dem Zementpulver darin evakuiert und die Monomerflüssigkeit durch die Leitung 72, das Anschlussstück 88, das Rohr der Rührwelle 53, den porösen Kern 75 und die Öffnungen 74 in den Mischraum 52 eingesaugt.

Die Sicherung wird herausgezogen beziehungsweise entfernt. Die Feder 66 treibt das Zahnrad 64 an, das Zahnrad 64 treibt das kleinere Zahnrad 65 (in den Abbildungen 4 und 5 oben) an und das größere Zahnrad 65 (in den Abbildungen 4 und 5 unten) treibt das Zahnrad 62 an der Rührwelle 53 an. Dadurch wird die Rührwelle 53 gedreht und bewegt sich bei der Drehung durch das Gleiten der Kurvenscheibe 86 auf dem abgeschrägten Stutzen im Gehäuse des Standfußes 58 auf und ab. Dabei drehen, heben und senken sich auch die Mischflügel 54 im Mischraum 52, wodurch die Ausgangskomponenten im Mischraum 52 durchmischt werden.

Nach erfolgter Durchmischung wird die Zementkartusche 51 mit dem gemischten Zementteig darin vom Standfuß 58 abgeschraubt und die Rührwelle 53 mit den Mischflügeln 54 daran aus der Austragsöffnung herausgezogen. In die Austragsöffnung wird ein Austragsrohr (nicht gezeigt) eingeschraubt, in dem ein zusätzlicher statischer Mischer enthalten sein kann, aber nicht enthalten sein muss. Die Arretierung des Austragskolbens mit der Innenwand der Zementkartusche 51 wird gelöst und der Austragskolben in Richtung der Austragsöffnung nach vorne getrieben. Dadurch wird der Inhalt des Mischraums 52 durch die Austragsöffnung ausgetrieben und der gemischte PMMA-Knochenzement kann so appliziert werden.

Die in der voranstehenden Beschreibung, sowie den Ansprüchen, Abbildungen und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 51: Zementkartusche
- 2, 52: Mischraum
- 3, 53: Rührwelle
- 4, 54: Mischflügel / Mischelement
- 6, 56: Stutzen
- 8, 58: Standfuß
- 10, 60: Dichtung
- 12, 62: Zahnrad der Rührwelle
- 14, 64: Zahnrad des Getriebes
- 16, 66: Feder / Spiralfeder
- 18: Sicherung
- 20, 70: Aufziehflügelstift
- 22, 72: Leitung
- 24, 74: Öffnung
- 25, 75: Poröser Kern
- 26, 76: Dichtungskolben
- 28, 78: Sterilisationskolben
- 30: Dichtung
- 32: Dichtung
- 34, 84: Vakuumschlauch
- 36, 86: Kurvenscheibe
- 38, 88: Anschlussstück
- 40: Sechskantloch
- 65: Zahnrad des Getriebes
- 67: Halterung
- 69: Ausnehmung

## Patentansprüche

1. Mischvorrichtung für Polymethylmethacrylat-Knochenzemente (PMMA-Knochenzemente) aufweisend
einen Mischraum (2, 52) zum Mischen des PMMA-Knochenzements,
zumindest ein in dem Mischraum (2, 52) drehbar gelagertes Mischelement (4, 54),
ein Getriebe, mit dem die Drehung des zumindest einen Mischelements (4, 54) anzutreiben ist,
mindestens ein elastisch verformbares Energiespeicherelement (16, 66), das mit dem Getriebe verbunden ist, so dass mit einer elastischen Energie aus dem Energiespeicherelement (16, 66) das Getriebe anzutreiben ist und bei Abgabe von elastischer Energie aus dem Energiespeicherelement (16, 66) das zumindest eine Mischelement (4, 54) über das Getriebe in dem Mischraum (2, 52) drehbar ist, **dadurch gekennzeichnet, dass**
die Bewegung des zumindest einen Mischelements (4, 54) über eine Kurvenscheibe (36, 86) bei der Drehbewegung zu beeinflussen ist, so dass das zumindest eine Mischelement (4, 54) während der Drehbewegung auch eine periodische axiale Hubbewegung durchführt, wobei mit der Kurvenscheibe (36, 86) die axiale Bewegung der Rührwelle (3, 53) zu erzeugen ist, so dass die Rührwelle (3, 53) während der Drehbewegung auch eine periodische axiale Hubbewegung durchführt.

2. Mischvorrichtung nach Anspruch 1, **dadurch gekennzeichnet, dass** das zumindest eine Mischelement (4, 54) auf einer drehbar gelagerten Rührwelle (3, 53) angeordnet ist und mit dem Getriebe eine Drehung der Rührwelle (3, 53) anzutreiben ist, wobei bevorzugt die Rührwelle (3, 53) durch eine gasdichte oder druckdichte Durchführung aus dem Mischraum (2, 52) herausgeführt ist und außerhalb des Mischraums (2, 52) mit dem Getriebe verbunden ist.

3. Mischvorrichtung nach Anspruch 2, **dadurch gekennzeichnet, dass**
die Rührwelle (3, 53) hohl ist und eine Leitung für eine flüssige Ausgangskomponente des PMMA-Knochenzements bildet, bevorzugt eine Leitung für eine Monomerflüssigkeit als Ausgangskomponente des PMMA-Knochenzements bildet.

4. Mischvorrichtung nach einem der vorangehenden Ansprüche, **dadurch gekennzeichnet, dass**
das zumindest eine Mischelement (4, 54) eine Mehrzahl von Mischflügeln (4, 54) ist, wobei zumindest während der Drehung der Mischflügel (4, 54) im Mischraum (2, 52) sich die Mischflügel (4, 54) radial zur Drehachse in den Mischraum (2, 52) erstrecken und gegen eine Ebene senkrecht zur Drehachse geneigt sind.

5. Mischvorrichtung nach den Ansprüchen 4 und 2 oder 3, **dadurch gekennzeichnet, dass**
die Mischflügel (4, 54) an der Rührwelle (3, 53) befestigt oder zu befestigen sind, wobei die Mischflügel (4, 54) axial zueinander versetzt auf der Rührwelle (3, 53) angeordnet sind.

6. Mischvorrichtung nach Anspruch 5, **dadurch gekennzeichnet, dass**
die Mischflügel (4, 54) über ein Gelenk an der Rührwelle (3, 53) befestigt sind, so dass die Mischflügel (4, 54) sich an die Rührwelle (3, 53) anlegen lassen.

7. Mischvorrichtung nach Anspruch 5 oder 6, **dadurch gekennzeichnet, dass**
die Mischflügel (4, 54) lösbar an der Rührwelle (3, 53) befestigt sind, so dass die Mischflügel (4, 54) beim Herausziehen der Rührwelle (3, 53) aus dem Mischraum (2, 52) von der Rührwelle (3, 53) getrennt werden, insbesondere abgebrochen werden.

8. Mischvorrichtung nach einem der vorangehenden Ansprüche, **dadurch gekennzeichnet, dass**
das Getriebe und/oder das elastisch verformbare Energiespeicherelement (16, 66) durch zumindest eine lösbare mechanische Sicherung (18) derart blockiert ist, dass eine Abgabe der Energie aus dem Energiespeicherelement (16, 66) verhindert ist, wobei bevorzugt die zumindest eine mechanische Sicherung (18) eine Sperrklinke und/oder ein Sperrstift (18) ist.

9. Mischvorrichtung nach einem der vorangehenden Ansprüche, **dadurch gekennzeichnet, dass**
das Getriebe ein Zahnradgetriebe oder ein Reibradgetriebe oder ein Zugmittelgetriebe ist.

10. Mischvorrichtung nach einem der vorangehenden Ansprüche, **dadurch gekennzeichnet, dass**
das elastisch verformbare Energiespeicherelement (16, 66) ein Federelement (16, 66) ist, das mit dem Getriebe verbunden ist, wobei das Getriebe durch die Federkraft des gespannten Federelements (16, 66) anzutreiben ist, wobei bevorzugt das Federelement (16, 66) ein gespanntes oder spannbares Federelement (16, 66) ist, besonders bevorzugt eine Metallfeder (16, 66) ist, ganz besonders bevorzugt eine Stahlschenkelfeder, eine Stahlblattfeder oder eine Stahlspiralfeder (16, 66) ist.

11. Mischvorrichtung nach einem der vorangehenden Ansprüche, **dadurch gekennzeichnet, dass**
eine Abgabe von elastischer Energie aus dem Energiespeicherelement (16, 66) eine Drehbewegung des Getriebes bewirkt, das Getriebe eine Übersetzung von wenigstens 2:1 auf das zumindest eine Mischelement (4, 54) bewirkt, bevorzugt mit einer Übersetzung von wenigstens 5:1, besonders bevorzugt von mindestens 8:1 auf das zumindest eine Mischelement (4, 54) bewirkt.

12. Vakuummischsystem zum Mischen eines PMMA-Knochenzements aufweisend eine Mischvorrichtung nach einem der vorangehenden Ansprüche, ferner aufweisend
den Mischraum (2, 52), in dem ein Zementpulver enthalten ist,
einen Monomerbehälter, der mit einem fluiden Monomer gefüllt ist und
einem Leitungsmittel (22, 72), das den Monomerbehälter mit dem Mischraum (2, 52) flüssigkeitsdurchlässig verbindet oder mit dem der Monomerbehälter mit dem Mischraum (2, 52) flüssigkeitsdurchlässig verbindbar ist.

13. Vakuummischsystem nach Anspruch 12, **dadurch gekennzeichnet, dass**
das Vakuummischsystem einen Standfuß (8, 58) aufweist, an dem der Mischraum (2, 52) lösbar befestigt ist und an dem der Monomerbehälter befestigt ist, wobei das Leitungsmittel (36, 86) und das elastische Energiespeicherelement (16, 66) in oder an dem Standfuß (8, 58) angeordnet sind.

14. Vakuummischsystem nach einem der Ansprüche 12 oder 13, **dadurch gekennzeichnet, dass**
sich eine Antriebsachse (3, 53), insbesondere die Rührwelle (3, 53), durch eine Dichtung (10, 60) aus dem Mischraum (2, 52) heraus erstreckt und an dem außerhalb des Mischraums (2, 52) angeordneten Teil der Antriebsachse (3, 53), insbesondere der Rührwelle (3, 53), ein Zahnrad (12, 62) oder Reibrad angebracht ist, das mit dem Getriebe verbunden ist, bevorzugt mit einem Zahnrad (14, 65), Reibrad, Zahnriemen oder Reibriemen des Getriebes verbunden ist, wobei die Antriebsachse (3, 53), insbesondere die Rührwelle (3, 53), über das Zahnrad (14, 65) oder Reibrad anzutreiben ist.

15. Vakuummischsystem nach einem der Ansprüche 12 bis 14, **dadurch gekennzeichnet, dass**
der Mischraum (2, 52) ein Teil einer Zementkartusche (1, 51) ist, wobei die Zementkartusche (1, 51) einseitig mit einem Austragskolben oder einem Austragskolbensystem (26, 28, 76, 78) verschlossen ist, wobei der Austragskolben oder das Austragskolbensystem (26, 28, 76, 78) in dem Mischraum (2, 52) axial beweglich und zum Austreiben des fertig gemischten PMMA-Knochenzements vorgesehen ist, der damit gebildete Verschluss für die Partikel des Zementpulvers undurchlässig ist und für Gas, insbesondere für Ethylenoxid, durchlässig ist, wobei die Zementkartusche (1, 51) vor der Vermischung der Zementkomponenten derart Vakuumdicht verschließbar ist, dass ein durch Vakuumeinwirkung bewirkter Transfer der Monomerflüssigkeit in den Mischraum (2, 52) der Zementkartusche (1, 51) zum Zementpulver möglich ist.

16. Vakuummischsystem nach einem der Ansprüche 12 bis 15, **dadurch gekennzeichnet, dass**
eine Vorrichtung zur Vakuumerzeugung in das Vakuummischsystem integriert ist.

17. Verfahren zum Mischen eines Zements, insbesondere eines PMMA-Knochenzements, bei dem zumindest zwei Ausgangskomponenten des Zements in einem Mischraum (2, 52) mit zumindest einem Mischelement (4, 54) gemischt werden, wobei das zumindest eine Mischelement (4, 54) in dem Mischraum (2, 52) um eine Drehachse gedreht wird und dabei die Ausgangskomponenten im Mischraum (2, 52) mischt, wobei die Drehung des zumindest einen Mischelements (4, 54) über ein Getriebe angetrieben wird und die Energie zum Antrieb des Getriebes von einem elastisch verformten Energiespeicherelement (16, 66) entnommen wird, wobei sich das elastisch verformte Energiespeicherelement (16, 66) bei der Energieentnahme entspannt, wobei das Mischelement (4, 54) periodisch in axialer Richtung bewegt wird und dabei die Ausgangskomponenten im Mischraum (2, 52) mischt **dadurch gekennzeichnet, dass** die periodische axiale Bewegung des zumindest einen Mischelements (4, 54) über eine Kurvenscheibe (36, 86) und ein Getriebe angetrieben wird und die Energie zum Antrieb des Getriebes und der Bewegung der Kurvenscheibe (36, 86) von dem elastisch verformten Energiespeicherelement (16, 66) entnommen wird, wobei sich das elastisch verformte Energiespeicherelement (16, 66) bei der Energieentnahme entspannt.

18. Verfahren nach Anspruch 17, **dadurch gekennzeichnet, dass** das Verfahren mit einer Vorrichtung nach einem der Ansprüche 1 bis 16 durchgeführt wird.

19. Verfahren nach Anspruch 17 oder 18, **dadurch gekennzeichnet, dass** eine erste Ausgangskomponente pulverförmig und bereits in dem Mischraum (2, 52) enthalten ist und eine zweite Ausgangskomponente eine Flüssigkeit ist, die in den Mischraum (2, 52) eingeleitet wird, bevorzugt mit einem Vakuum in den Mischraum (2, 52) eingesaugt wird.

20. Verfahren nach einem der Ansprüche 17 bis 19, **dadurch gekennzeichnet, dass** nach dem Mischen des Zements eine Rührwelle (3, 53), an der das zumindest eine Mischelement (4, 54) in Form von Mischflügeln (4, 54) befestigt ist, aus dem Mischraum (2, 52) herausgezogen wird, wobei die Mischflügel (4, 54) abbrechen und im Mischraum (2, 52) verbleiben oder die Mischflügel (4, 54) sich an die Rührwelle (3, 53) anlegen und mit der Rührwelle (3, 53) aus dem Mischraum (2, 52) herausgezogen werden.

21. Verfahren nach einem der Ansprüche 17 bis 20, **dadurch gekennzeichnet, dass** das Energiespeicherelement (16, 66) eine gespannte Metallfeder (16, 66) ist, die sich bei der Abgabe von elastischer Energie an das Getriebe entspannt und dadurch das Getriebe angetrieben wird.

22. Verfahren nach einem der Ansprüche 17 bis 21, **dadurch gekennzeichnet, dass** die elastische Energie aus dem Energiespeicherelement (16, 66) entnommen wird, indem eine mechanische Sicherung (18) gelöst wird, die das Getriebe und/oder das elastisch verformbare Energiespeicherelement (16, 66) blockiert, wobei nach dem Lösen die elastische Energie von dem Energiespeicherelement (16, 66) an das Getriebe abgegeben wird, wobei bevorzugt eine Sperrklinke und/oder ein Sperrstift (18) als mechanische Sicherung (18) verwendet wird, die besonders bevorzugt in das Getriebe eingreift und das Getriebe arretiert.

## Claims

1. Mixing device for polymethylmethacrylate bone cements (PMMA bone cements) comprising
a mixing space (2, 52) for mixing the PMMA bone cement;
at least one mixing element (4, 54) that is supported in the mixing space (2, 52) such that it can be rotated;
a gear by means of which the rotation of the at least one mixing element (4, 54) can be driven;
at least one energy storage element (16, 66) that can be deformed elastically and is connected to the gear such that the gear can be driven by an elastic energy from the energy storage element (16, 66) and such that the at least one mixing element (4, 54) can be rotated in the mixing space (2, 52) by means of the gear upon the release of elastic energy from the energy storage element (16, 66); **characterised in that**
the motion of the at least one mixing element (4, 54) during the rotary motion can be influenced by a cam disk (36, 86) such that the at least one mixing element (4, 54) also performs a periodical axial lift motion during the rotary motion, whereby the axial motion of the agitator shaft (3, 53) can be generated by the cam disk (36, 86) such that the agitator shaft (3, 53) also performs a periodical axial lift motion during the rotary motion.

2. Mixing device according to claim 1, **characterised in that**
the at least one mixing element (4, 54) is arranged on an agitator shaft (3, 53), which is supported such that it can be rotated, and a rotation of the agitator shaft (3, 53) can be driven by means of the gear, whereby the agitator shaft (3, 53) preferably is guided out of the mixing space (2, 52) through a gas-tight or pressure-type feed-through and is connected to the gear outside of the mixing space (2, 52).

3. Mixing device according to claim 2, **characterised in that** the agitator shaft (3, 53) is hollow and forms a conduit for a liquid starting component of the PMMA bone cement, preferably forms a conduit for a monomer liquid as starting component of the PMMA bone cement.

4. Mixing device according to any one of the preceding claims, **characterised in that**
the at least one mixing element (4, 54) is a plurality of mixing vanes (4, 54), whereby, at least during the rotation of the mixing vanes (4, 54) in the mixing space (2, 52), the mixing vanes (4, 54) extend radially, with respect to the rotary axis, into the mixing space (2, 52) and are inclined with respect to a plane that is perpendicular to the rotary axis.

5. Mixing device according to claims 4 and 2 or 3, **characterised in that**
the mixing vanes (4, 54) are or can be attached to the agitator shaft (3, 53), whereby the mixing vanes (4, 54) are arranged on the agitator shaft (3, 53) such as to be axially offset with respect to each other.

6. Mixing device according to claim 5, **characterised in that**
the mixing vanes (4, 54) are attached to the agitator shaft (3, 53) by means of a joint such that the mixing vanes (4, 54) can be folded against the agitator shaft (3, 53).

7. Mixing device according to claim 5 or 6, **characterised in that**
the mixing vanes (4, 54) are attached to the agitator shaft (3, 53) in detachable manner such that the mixing vanes (4, 54) are severed, in particular are broken off, from the agitator shaft (3, 53), when the agitator shaft (3, 53) is pulled out of the mixing space (2, 52).

8. Mixing device according to any one of the preceding claims, **characterised in that**
the gear and/or the energy storage element (16, 66) that can be deformed elastically are blocked through at least one detachable mechanical securing facility (18) such that a release of energy from the energy storage element (16, 66) is prevented, whereby the at least one mechanical securing facility (18) is preferred to be a safety catch and/or a safety pin (18).

9. Mixing device according to any one of the preceding claims, **characterised in that**
the gear is a cogwheel gear or a friction gear or a traction gear.

10. Mixing device according to any one of the preceding claims, **characterised in that**
the energy storage element (16, 66) that can be deformed elastically is a spring element (16, 66) that is connected to the gear, whereby the gear can be driven through the spring force of the tensioned spring element (16, 66), whereby the spring element (16, 66) preferably is a spring element (16, 66) that is or can be tensioned, particularly preferably is a metal spring (16, 66), even more particularly preferably is a steel leg spring, a steel leaf spring or a steel coil spring (16, 66).

11. Mixing device according to any one of the preceding claims, **characterised in that**
a release of elastic energy from the energy storage element (16, 66) effects a rotary motion of the gear, the gear effects a transmission of at least 2:1 to the at least one mixing element (4, 54), preferably with a transmission of at least 5:1, particularly preferably of at least 8:1 to the at least one mixing element (4, 54).

12. Vacuum mixing system for mixing a PMMA bone cement comprising a mixing device according to any one of the preceding claims, further comprising
the mixing space (2, 52) that contains a cement powder; a monomer container that is filled with a fluid monomer; and
a conduit means (22, 72) that connects the monomer container to the mixing space (2, 52) in a liquid-permeable manner or by means of which the monomer container can be connected to the mixing space (2, 52) in liquid-permeable manner.

13. Vacuum mixing system according to claim 12, **characterised in that**
the vacuum mixing system comprises a stand (8, 58) to which the mixing space (2, 52) is attached in detachable manner and to which the monomer container is attached, whereby the conduit means (36, 86_{[A1]}) and the elastic energy storage element (16, 66) are arranged in or on the stand (8, 58).

14. Vacuum mixing system according to any one of the claims 12 or 13,
**characterised in that**
a drive axle (3, 53), in particular the agitator shaft (3, 53), extends out of the mixing space (2, 52) through a feed-through (10, 60) and **in that** a gear wheel (12, 62) or friction wheel is attached to the part of the drive axle (3, 53), in particular the agitator shaft (3, 53), that is arranged outside of the mixing space (2, 52), and is connected to the gear, preferably is connected to a gear wheel (14, 65), friction wheel, toothed belt or friction belt of the gear, whereby the drive axle (3, 53), in particular the agitator shaft (3, 53), can be driven by means of the gear wheel (14, 65) or friction wheel.

15. Vacuum mixing system according to any one of the claims 12 to 14,
**characterised in that**
the mixing space (2, 52) is a part of a cement cartridge (1, 51), whereby the cement cartridge (1, 51) is closed on one side by a dispensing plunger or a dispensing plunger system (26, 28, 76, 78), whereby the dispensing plunger or the dispensing plunger system (26, 28, 76, 78) is provided to be axially mobile in the mixing space (2, 52) and is provided for expelling the ready-mixed PMMA bone cement, [whereby] the closure thus formed is impermeable to the particles of the cement powder and is permeable for gas, in particular for ethylene oxide, whereby the cement cartridge (1, 51) can be closed appropriately in vacuum-tight manner before the cement components are being mixed such that a transfer of the monomer liquid into the mixing space (2, 52) of the cement cartridge (1, 51) to the cement powder due to the effect of vacuum is feasible.

16. Vacuum mixing system according to any one of the claims 12 to 15,
**characterised in that**
a device for production of a vacuum is integrated into the vacuum mixing system.

17. Method for mixing a cement, in particular a PMMA bone cement, in which at least two starting components of the cement are mixed in a mixing space (2, 52) with at least one mixing element (4, 54), whereby the at least one mixing element (4, 54) is being rotated about a rotary axis in the mixing space (2, 52) and, in the process, mixes the starting components in the mixing space (2, 52), whereby the rotation of the at least one mixing element (4, 54) is driven by means of a gear and the energy for driving the gear is taken from an elastically deformed energy storage element (16, 66), whereby the elastically deformed energy storage element (16, 66) relaxes upon energy being taken out, whereby the mixing element (4, 54) is periodically moved in axial direction and, in the process, mixes the starting components in the mixing space (2, 52), **characterised in that** the periodical axial motion of the at least one mixing element (4, 54) is driven by means of a cam disk (36, 86) and a gear and **in that** the energy for driving the gear and the motion of the cam disk (36, 86) is taken from the elastically deformed energy storage element (16, 66), whereby the elastically deformed energy storage element (16, 66) relaxes upon energy being taken out.

18. Method according to claim 16, **characterised in that**
the method is implemented with a device according to any one of the claims 1 to 16.

19. Method according to claim 17 or 18, **characterised in that**
a first starting component is powdered and is already contained in the mixing space (2, 52) and **in that** a second starting component is a liquid that is being guided into the mixing space (2, 52), preferably is being aspirated into the mixing space (2, 52) by means of a vacuum.

20. Method according to any one of the claims 17 to 19, **characterised in that** after the cement being mixed, an agitator shaft (3, 53) to which the at least one mixing element (4, 54), in the form of mixing vanes (4, 54), is attached, is pulled out of the mixing space (2, 52), whereby the mixing vanes (4, 54) break off and remain in the mixing space (2, 52) or the mixing vanes (4, 54) fold against the agitator shaft (3, 53) and are pulled out of the mixing space (2, 52) along with the agitator shaft (3, 53).

21. Method according to any one of the claims 17 to 20, **characterised in that**
the energy storage element (16, 66) is a tensioned metal spring (16, 66) that relaxes upon the release of elastic energy to the gear, and **in that** the gear is driven by this means.

22. Method according to any one of the claims 17 to 21, **characterised in that**
the elastic energy is taken from the energy storage element (16, 66) by detaching a mechanical securing facility (18) that blocks the gear and/or the energy storage element (16, 66) that can be deformed elastically, whereby, after detachment, the elastic energy is released from the energy storage element (16, 66) to the gear, whereby a safety catch and/or a safety pin (18), which particularly preferably engages the gear or arrests the gear, is preferably used as the mechanical securing facility (18).

## Revendications

1. Dispositif de mélange pour ciments osseux de polyméthylméthacrylate (ciments osseux de PMMA) présentant
un espace de mélange (2, 52) pour le mélange du ciment osseux de PMMA, au moins un élément de mélange (4, 54) logé à rotation dans l'espace de mélange (2, 52),
un engrenage avec lequel la rotation de l'au moins un élément de mélange (4, 54) est à entraîner,
au moins un élément d'accumulation d'énergie déformable élastiquement (16, 66) qui est relié à l'engrenage de sorte que l'engrenage est à entraîner avec une énergie élastique provenant de l'élément d'accumulation d'énergie (16, 66) et l'au moins un élément de mélange (4, 54) peut être tourné par le biais de l'engrenage dans l'espace de mélange (2, 52) lors du transfert d'énergie élastique provenant de l'élément d'accumulation d'énergie (16, 66),
**caractérisé en ce que**
le mouvement de l'au moins un élément de mélange (4, 54) est à influencer par le biais d'une came (36, 86) lors du mouvement de rotation de sorte que l'au moins un élément de mélange (4, 54) réalise pendant le mouvement de rotation également un mouvement de levage axial périodique, dans lequel le mouvement axial de l'arbre d'agitation (3, 53) est à générer avec la came (36, 86) de sorte que l'arbre d'agitation (3, 53) réalise pendant le mouvement de rotation également un mouvement de levage axial périodique.

2. Dispositif de mélange selon la revendication 1, **caractérisé en ce que**
l'au moins un élément de mélange (4, 54) est disposé sur un arbre d'agitation logé à rotation (3, 53) et une rotation de l'arbre d'agitation (3, 53) est à entraîner avec l'engrenage, dans lequel l'arbre d'agitation (3, 53) est de préférence guidé hors de l'espace de mélange (2, 52) à travers un passage étanche au gaz ou étanche à la pression et est relié à l'engrenage en dehors de l'espace de mélange (2, 52).

3. Dispositif de mélange selon la revendication 2, **caractérisé en ce que** l'arbre d'agitation (3, 53) est creux et forme une conduite pour un composant de départ liquide du ciment osseux de PMMA, forme de préférence une conduite pour un liquide monomère en tant que composant de départ du ciment osseux de PMMA.

4. Dispositif de mélange selon une des revendications précédentes, **caractérisé en ce que**
l'au moins un élément de mélange (4, 54) est une pluralité de pales de mélange (4, 54), dans lequel les pales de mélange (4, 54) s'étendent radialement par rapport à l'axe de rotation dans l'espace de mélange (2, 52) au moins pendant la rotation des pales de mélange (4, 54) dans l'espace de mélange (2, 52) et sont inclinées contre un plan perpendiculaire à l'axe de rotation.

5. Dispositif de mélange selon les revendications 4 et 2 ou 3, **caractérisé en ce que**
les pales de mélange (4, 54) sont fixées ou à fixer à l'arbre d'agitation (3, 53), dans lequel les pales de mélange (4, 54) sont disposées sur l'arbre d'agitation (3, 53) de manière décalée axialement les unes des autres.

6. Dispositif de mélange selon la revendication 5, **caractérisé en ce que**
les pales de mélange (4, 54) sont fixées à l'arbre d'agitation (3, 53) par le biais d'une articulation de sorte que les pales de mélange (4, 54) peuvent être appliquées contre l'arbre d'agitation (3, 53).

7. Dispositif de mélange selon la revendication 5 ou 6, **caractérisé en ce que**
les pales de mélange (4, 54) sont fixées à l'arbre d'agitation (3, 53) de manière amovible de sorte que les pales de mélange (4, 54) sont séparées de l'arbre d'agitation (3, 53) lors de l'extraction de l'arbre d'agitation (3, 53) de l'espace de mélange (2, 52), sont notamment démontées.

8. Dispositif de mélange selon une des revendications précédentes, **caractérisé en ce que**
l'engrenage et/ou l'élément d'accumulation d'énergie déformable élastiquement (16, 66) est bloqué par au moins une fixation mécanique amovible (18) de telle sorte qu'un transfert de l'énergie provenant de l'élément d'accumulation d'énergie (16, 66) est empêché, dans lequel l'au moins une fixation mécanique (18) est de préférence un cliquet d'arrêt et/ou une broche d'arrêt (18).

9. Dispositif de mélange selon une des revendications précédentes, **caractérisé en ce que**
l'engrenage est un engrenage à roue dentée ou un engrenage à roue de friction ou un engrenage à moyen de traction.

10. Dispositif de mélange selon une des revendications précédentes, **caractérisé en ce que**
l'élément d'accumulation d'énergie déformable élastiquement (16, 66) est un élément de ressort (16, 66) qui est relié à l'engrenage, dans lequel l'engrenage est à entraîner par la résilience de l'élément de ressort tendu (16, 66), dans lequel l'élément de ressort (16, 66) est de préférence un élément de ressort tendu ou pouvant être tendu (16, 66), est de manière particulièrement préférée un ressort métallique (16, 66), est de manière tout particulièrement préférée un ressort à branches en acier, un ressort à lames en acier ou un ressort à spirale en acier (16, 66).

11. Dispositif de mélange selon une des revendications précédentes, **caractérisé en ce que**
un transfert d'énergie élastique provenant de l'élément d'accumulation d'énergie (16, 66) entraîne un mouvement de rotation de l'engrenage, l'engrenage entraîne une translation d'au moins 2/1 sur l'au moins un élément de mélange (4, 54), entraîne de préférence avec une translation d'au moins 5/1, de manière particulièrement préférée d'au moins 8/1 sur l'au moins un élément de mélange (4, 54).

12. Système de mélange sous vide pour le mélange d'un ciment osseux de PMMA présentant un dispositif de mélange selon une des revendications précédentes, présentant en outre
l'espace de mélange (2, 52) dans lequel une poudre de ciment est contenue, un récipient de monomère qui est rempli d'un monomère fluide et
un moyen de conduite (22, 72) qui relie de manière perméable au liquide le récipient de monomère à l'espace de mélange (2, 52) ou avec lequel le récipient de monomère peut être relié de manière perméable au liquide à l'espace de mélange (2, 52).

13. Système de mélange sous vide selon la revendication 12, **caractérisé en ce que**
le système de mélange sous vide présente un pied (8, 58) auquel l'espace de mélange (2, 52) est fixé de manière amovible et auquel le récipient de monomère est fixé, dans lequel le moyen de conduite (36, 86) et l'élément d'accumulation d'énergie élastique (16, 66) sont disposés dans ou sur le pied (8, 58).

14. Système de mélange sous vide selon une des revendications 12 ou 13,
**caractérisé en ce que**
un arbre primaire (3, 53), notamment l'arbre d'agitation (3, 53), s'étend à travers un joint (10, 60) hors de l'espace de mélange (2, 52) et une roue dentée (12, 62) ou roue de friction qui est reliée à l'engrenage, est de préférence reliée à une roue dentée (14, 65), roue de friction, courroie dentée ou courroie de friction de l'engrenage, est montée sur la partie de l'arbre primaire (3, 53), notamment de l'arbre d'agitation (3, 53), disposée en dehors de l'espace de mélange (2, 52), dans lequel l'arbre primaire (3, 53), notamment l'arbre d'agitation (3, 53), est à entraîner par le biais de la roue dentée (14, 65) ou roue de friction.

15. Système de mélange sous vide selon une des revendications 12 à 14,
**caractérisé en ce que**
l'espace de mélange (2, 52) fait partie d'une cartouche de ciment (1, 51), dans lequel la cartouche de ciment (1, 51) est fermée d'un côté avec un piston d'extraction ou un système de piston d'extraction (26, 28, 76, 78), dans lequel le piston d'extraction ou le système de piston d'extraction (26, 28, 76, 78) est mobile axialement dans l'espace de mélange (2, 52) et prévu pour l'expulsion du ciment osseux de PMMA entièrement mélangé, la fermeture ainsi formée est imperméable pour les particules de la poudre de ciment et est perméable pour du gaz, notamment pour de l'oxyde d'éthylène, dans lequel la cartouche de ciment (1, 51) peut être fermée de manière étanche au vide avant le mélange des composants de ciment de telle sorte qu'un transfert du liquide monomère entraîné par une action du vide dans l'espace de mélange (2, 52) de la cartouche de ciment (1, 51) vers la poudre de ciment est possible.

16. Système de mélange sous vide selon une des revendications 12 à 15,
**caractérisé en ce que**
un dispositif pour la génération de vide est intégré dans le système de mélange sous vide.

17. Procédé pour le mélange d'un ciment, notamment d'un ciment osseux de PMMA, lors duquel au moins deux composants de départ du ciment sont mélangés dans un espace de mélange (2, 52) avec au moins un élément de mélange (4, 54), dans lequel l'au moins un élément de mélange (4, 54) est tourné dans l'espace de mélange (2, 52) autour d'un axe de rotation et mélange ce faisant les composants de départ dans l'espace de mélange (2, 52), dans lequel la rotation de l'au moins un élément de mélange (4, 54) est entraînée par le biais d'un engrenage et l'énergie pour l'entraînement de l'engrenage est prélevée d'un élément d'accumulation d'énergie déformé élastiquement (16, 66), dans lequel l'élément d'accumulation d'énergie déformé élastiquement (16, 66) se détend lors du prélèvement d'énergie, dans lequel l'élément de mélange (4, 54) est déplacé périodiquement dans la direction axiale et mélange ce faisant les composants de départ dans l'espace de mélange (2, 52), **caractérisé en ce que** le mouvement axial périodique de l'au moins un élément de mélange (4, 54) est entraîné par le biais d'une came (36, 86) et d'un engrenage et l'énergie pour l'entraînement de l'engrenage et du mouvement de la came (36, 86) est prélevée de l'élément d'accumulation d'énergie déformé élastiquement (16, 66), dans lequel l'élément d'accumulation d'énergie déformé élastiquement (16, 66) se détend lors du prélèvement d'énergie.

18. Procédé selon la revendication 17, **caractérisé en ce que**
le procédé est réalisé avec un dispositif selon une des revendications 1 à 16.

19. Procédé selon la revendication 17 ou 18, **caractérisé en ce que**
un premier composant de départ est pulvérulent et déjà contenu dans l'espace de mélange (2, 52) et un second composant de départ est un liquide qui est introduit dans l'espace de mélange (2, 52), est de préférence aspiré avec un vide dans l'espace de mélange (2, 52).

20. Procédé selon une des revendications 17 à 19, **caractérisé en ce que**
après le mélange du ciment, un arbre d'agitation (3, 53) auquel l'au moins un élément de mélange (4, 54) sous forme de pales de mélange (4, 54) est fixé est extrait de l'espace de mélange (2, 52), dans lequel les pales de mélange (4, 54) se démontent et restent dans l'espace de mélange (2, 52) ou les pales de mélange (4, 54) s'appliquent contre l'arbre d'agitation (3, 53) et sont extraites de l'espace de mélange (2, 52) avec l'arbre d'agitation (3, 53).

21. Procédé selon une des revendications 17 à 20, **caractérisé en ce que**
l'élément d'accumulation d'énergie (16, 66) est un ressort métallique tendu (16, 66) qui se détend lors du transfert d'énergie élastique à l'engrenage et l'engrenage est entraîné ce faisant.

22. Procédé selon une des revendications 17 à 21, **caractérisé en ce que**
l'énergie élastique est prélevée de l'élément d'accumulation d'énergie (16, 66) **en ce qu'**une fixation mécanique (18) est desserrée, laquelle bloque l'engrenage et/ou l'élément d'accumulation d'énergie déformable élastiquement (16, 66), dans lequel, après le desserrage, l'énergie élastique de l'élément d'accumulation d'énergie (16, 66) est transférée à l'engrenage, dans lequel un cliquet d'arrêt et/ou une broche d'arrêt (18) est de préférence utilisé(e) en tant que fixation mécanique (18), laquelle s'engrène de manière particulièrement préférée dans l'engrenage et arrête l'engrenage.
